# EUROPÄISCHE PATENTSCHRIFT

(11) **EP 1 948 819 B1**
(45) Veröffentlichungstag und Bekanntmachung des Hinweises auf die Patenterteilung: **15.04.2009**
(21) Anmeldenummer: 06804394.2
(22) Anmeldetag: 17.11.2006
(51) Int. Cl.: C12Q 1/26, G01N 33/84

(54) **BESTIMMUNG VON DIAMINOOXIDASE**
DIAMINE OXIDASE DETERMINATION
DETERMINATION DE DIAMINOOXYDASE

(30) Priorität: 18.11.2005 AT 18812005
(43) Veröffentlichungstag der Anmeldung: 30.07.2008
(73) Patentinhaber: Sciotec Diagnostic Technologies GmbH, 1110 Wien (AT)
(72) Erfinder: MAYER, Isabella, A-3660 Klein Pöchlarn (AT); MISSBICHLER, Albert, A-1210 Wien (AT)
(74) Vertreter: Sonn & Partner Patentanwälte
(86) Internationale Anmeldenummer: PCT/AT2006/000473
(87) Internationale Veröffentlichungsnummer: WO 2007/056788

(56) Entgegenhaltungen:
- AT-B- 411 688
- US-A1- 2005 214 894
- MOREL A M ET AL: "Immunoanalysis of histamine through a novel chemical derivatization." THE JOURNAL OF ALLERGY AND CLINICAL IMMUNOLOGY OCT 1988, Bd. 82, Nr. 4, Oktober 1988 (1988-10), Seiten 646-654, XP002423996 ISSN: 0091-6749

## Beschreibung

Die Erfindung betrifft ein Verfahren zur Bestimmung der Aktivität von Diaminooxdase (DAO; EC 1.4.3.6).

Histamin (1H-Imidazol-4-Ethylamin) entsteht durch enzymatische Decarboxylierung von Histidin und ist somit ein basisches biogenes Amin mit einem Molekulargewicht von 111 Da.

Im Organismus kommt Histamin praktisch ubiquitär vor. Es wird vom Menschen selbst produziert und in den metachromatischen Granula der Mastzellen und basophilen Leukozyten gespeichert, wo es zur sofortigen Freisetzung zur Verfügung steht. Die höchsten Histamin-Konzentrationen werden in der Lunge gemessen. Nach der Freisetzung ist Histamin ein äußerst potenter Mediator einer Vielzahl von physiologischen und pathophysiologischen Prozessen, oftmals auch über Wechselwirkung mit Zytokinen.

Darüber hinaus kann Histamin auch von außen in den Körper gelangen, einerseits durch Einatmen oder aber auf dem oralen Weg, beispielsweise durch die Aufnahme von histaminhältigen Lebensmitteln, wie Käse, Wein, Fischkonserven und Sauerkraut.

Die wichtigsten Funktionen und Wirkungen von Histainin im menschlichen bzw. tierischen Körper sind:
1.Dilatation der Kapillaren, Erhöhung der Kapillarpermeabilität und Blutdruckabfall.
2.Kontraktionen der glatten Muskulatur, u.a. der Bronchialmuskeln in der Lunge.
3.Induktion einer erhöhten Magensäuresekretion.
4.Erhöhung der Herzfrequenz.
5.Histamin ist der Mediator der allergischen Soforttyp-Reaktion, es ist der wichtigste Mediator bei allergischen Erkrankungen, wie Rhinitis allergica (Heuschnupfen) und Asthma bronchiale.
6.Darüber hinaus ist Histamin der klassische Auslöser einer Urticaria (Nesselausschlag) und spielt bei Medikamenten-Allergien bzw. Unverträglichkeiten eine wichtige Rolle.

Erhöhte Konzentrationen von freiem Histamin im Blutkreislauf einerseits oder im Darmlumen andererseits lösen unerwünschte Wirkungen aus, wie Kopfschmerzen, verlegte bzw. rinnende Nase, Atemwegsobstruktionen, Tachykardie sowie Extrasystolen, weiters Magen- und Darmbeschwerden, die zu weichem Stuhl bis Durchfällen führen können, und Hypotonie. Oft werden auch Schwellungen der Augenlider, gelegentlich auch urticarielle Exantheme beschrieben. Des Weiteren können Hautrötungen, Blutdruckabfall und Bronchospasmen auftreten.

Im Organismus von Säugetieren wird Histamin von zwei Enzymen abgebaut: Diaminooxidase (DAO, EC 1.4.3.6) und Histamin-N-Methyltransferase (NMT, EC 2.1.1.8) (Mizuguchi et al. 1994). DAO katalysiert die oxidative Deaminierung von Histamin zu Imidazolazetaldehyd; NMT katalysiert die N-Methylierung zu N-Methylhistamin.

Beide Abbauwege sind für den Organismus essentiell: DAO entfernt Histamin, das beispielsweise über die Nahrung in den Gastrointestinaltrakt aufgenommen wurde, NMT steuert die histaminerge Signalübertragung im Nervensystem (Kitanaka et al. 2002).

Die Hauptaufgabe der DAO ist es zu verhindern, dass über die Nahrung aufgenommenes Histamin aus dem Darm in den Blutkreislauf gelangt oder direkt ins vegetative Nervensystem aufgenommen wird. Versagt dieser Schutzmechanismus, kann es im Extremfall auch zu einem anapyhlaktischen Schock kommen (Taylor 1986, Nilsson et al. 1996). DAO ist ein sekretorisches Protein und arbeitet daher extrazellulär, wohingegen N-Methyltransferase ausschließlich im Zytosol aktiv ist (Küfner et al. 2001).

Die native DAO, die neben Histamin weitere biogene Amine, wie beispielsweise Putrescin, Spermidin und Kadaverin, abbauen kann, und die beispielsweise aus Schweineniere gewonnen wird, ist ein homodimeres kupferhältiges Glycoprotein, bei welchem die Untereinheiten über Disulfidbrücken verbunden sind. DAO hat ein Molekulargewicht von etwa 182 kDa (Kluetz and Schmidt 1997, Rinaldi et al. 1982) und einen Kohlenhydratanteil von etwa 11% (Shah and Ali 1988). Das Enzym gehört zur Klasse der kupferhältigen Aminooxidasen, welche die oxidative Deaminierung von primären Aminen zu Aldehyden, Ammoniak und Wasserstoffperoxid nach dem folgenden allgemeinen Reaktionsschema katalysieren (Bachrach 1985) : RCH₂NH₂ + H₂O + O₂ => RCHO + NH₃ + H₂O₂, wobei der Rest R eine Aminogruppe enthält.

Charakteristisch für die kupferhältigen Aminooxidasen ist ein Topachinon im aktiven Zentrum, das durch post-translationale Modifikation eines konservierten Tyrosin-Rests entsteht (James et al. 1990, James et al. 1992, Mu et al. 1992).

DAO ist hauptsächlich im Dünndarm, in der Leber, in den Nieren und im Blut in weißen Blutzellen zu finden. Bei Schwangeren wird DAO zusätzlich in der Plazenta gebildet. Schwangere haben etwa 500 bis 1000 mal höhere Blut-DAO-Spiegel als Nicht-Schwangere.

DAO wird kontinuierlich produziert und in das Darmlumen ausgeschieden. Bei einem gesunden Menschen wird Histamin-reiche Nahrung daher bereits im Darm von Histamin weitgehend befreit. Das verbleibende Histamin wird beim Durchtritt durch die Darmschleimhaut von der dort sitzenden DAO abgebaut. Histamin wird zu Imidazolacetaldehyd und weiters zu Imidazolacetessigsäure zerlegt. Die Co-Faktoren der Diaminooxidase sind 6-Hydroxydopa und wahrscheinlich Pyridoxalphosphat, das Vitamin B6. Diaminooxidase ist ein empfindliches Enzym, das von verschiedenen Substanzen, wie anderen biogenen Aminen, Alkohol und seinem Abbauprodukt Acetaldehyd und verschiedenen Medikamenten, gehemmt werden kann.

Wie bereits zuvor erwähnt kann über die Nahrung aufgenommenes, körperfremdes Histamin aber auch körpereigenes Histamin aufgrund von allergischen Reaktionen eine Vielfalt von Störungen auslösen. Hinsichtlich der klinischen Wertigkeit sind mindestens drei Formen einer Histamin-Intoleranz auf der Basis einer verminderten Diaminooxidaseaktivität hervorzuheben:
- Wenige Menschen haben einen angeborenen DAO-Mangel und verlieren diesen auch nicht.
- Während eines Infektes der Darmschleimhaut kann ein vorübergehender DAO-Mangel auftreten. Nach Abheilen des Infektes normalisiert sich auch die DAO-Aktivität.
- Im Rahmen der Gabe verschiedener aktivitätshemmender Substanzen kann es zu einer exogen vermittelten verminderten DAO-Aktivität kommen. Dazu gehören vorrangig Alkohol und sein Abbauprodukt Acetaldehyd, gewisse aminreiche Nahrungsmittel und viele Medikamente.

In allen Fällen treten die eingangs beschriebenen Symptome mehr oder weniger massiv auf und lassen sich in den meisten Fällen nicht einfach zuordnen. Eine schnelle Abklärung der funktionellen Aktivität des Enzyms erlaubt eine rasche und einfache Therapie und die Erstellung eines entsprechenden Diätplans.

Die US 5 284 749 offenbart ein Verfahren zur Bestimmung von Diaminooxidase in einer Probe, bei dem zur Bestimmung der Diaminooxidase Antikörper verwendet werden. Insbesondere dient das in der US-Schrift beschriebene Verfahren zur Unterscheidung zwischen Diaminooxidase aus dem Fruchtwasser und Diaminooxidase aus dem Serum.

In der US 5 124 254 ist ein Verfahren zur Detektion von Putrescin und Cadaverin beschrieben, bei dem eine Probe mit Diaminooxidase umgesetzt wird und das dabei gebildete Wasserstoffperoxid mittels einer weiteren Reaktion, die zu einer Farbbildung führt, detektiert wird.

Die Methodik des Nachweises der Enzymaktivität im Plasma beruht auf der Eigenschaft der DAO, die Oxidation von Diaminen, wie Histamin, Putrescin (1,4-Butandiamin) und Kadaverin (1,5-Pentandiamin), zu katalysieren.

Eine weit verbreitete Standard-Methode zur Bestimmung der DAO-Aktivität beruht auf der Umsetzung von ¹⁴C-markiertem Putrescin oder Kadaverin mit DAO. Der dabei gebildete Monoaldehyd zyklisiert intramolekular mit der Aminogruppe und kann aufgrund der schlechteren Wasserlöslichkeit mit z.B. Chloroform (CHCl₃) extrahiert werden. Anschließend erfolgt die Aktivitätsbestimmung mittels Flüssigszintillationszählung (Tufvesson G; Tryding N: "Determination of diamine oxidase activity in normal human blood serum", Scand J Clin Lab Invest 1969 Sep;24(2):163-8(ISSN: 0036-5513)).

Diese Methodik weist, obwohl nahezu universell angewendet, essentielle Schwachstellen auf, die bislang einer weiteren Verbreitung der Anwendung dieses Prinzips im Wege standen: Beispielsweise sind die eingesetzten Reagentien als giftig bzw. gesundheitsschädlich einzustufen. Um die Extraktionsausbeute zu verbessern, wurde in einer derzeit angewandten Methode Chloroform (mutagen, krebserregend) durch Toluol (gesundheitsschädlich, leicht entzündlich, fortpflanzungsgefährdend, fruchtschädigend) ersetzt. Auch dieses Reagenz ist daher nicht unbedenklich. Zudem muss das Gemisch aus Plasma und Toluol gefroren werden, um eine brauchbare Trennung von der dann festen Plasmaphase und der flüssigen Toluolphase zu ermöglichen. Weiters ist auch die Reaktivität des Enzyms relativ schwach, so dass mindestens 250 µl Probe eingesetzt werden müssen, was eine entsprechend große Menge an Extraktionsmittel bedingt.

Ein weiteres Verfahren zur Bestimmung der Enzymaktivität von DAO ist in der AT 411688 offenbart. Das darin beschriebene Verfahren erfordert zunächst das Umsetzen einer wässerigen Lösung einer bestimmten Menge eines Diamins (z.B. Histamin, Putrescin, Kadaverin), das intramolekular zyklisiert, nachdem eine der Amingruppen von DAO zu einer Aldehydgruppe umgewandelt wird, wobei das zyklisierte Molekül in Ethylacetat eine höhere Löslichkeit aufweist als das nicht zyklisierte Diamin, mit DAO, wobei eine der Aminogruppen zu einer Aldehydgruppe oxidiert wird und diese Aldehydgruppe intramolekular mit der zweiten Amingruppe zyklisiert. Anschließend wird das zyklisierte Produkt mit Ethylacetat extrahiert und detektiert. Das Diamin wird vorzugsweise radioaktiv, insbesondere mit ¹³C oder ¹⁴C, oder mit einem Farbstoff oder einer chromogenen Gruppe markiert, insbesondere mit einem Fluoreszenz-Farbstoff oder einem Fluorogen.

Ferner sind in der wissenschaftlichen Literatur Verfahren zur Bestimmung der DAO Aktivität beschrieben, die das bei der DAO-katalysierten Reaktion von Diamin mit Wasser und Sauerstoff erzeugte Wasserstoffperoxid messen. In Kohoe CA et al. (J Nutr (2000) 130:30-33), beispielsweise, wird die DAO-Aktivität durch die Umsetzung von Kadaverin Dihydrochlorid bestimmt. Das bei der Reaktion von DAO mit Kadaverin gebildete Wasserstoffperoxid wird anschließend mit dem Natrium-Salz von 10-(Carboxymethylaminocarbonyl)-3,7-bis-(Dimethylamino)-Phenothiazin, Ascorbatoxidase und Meerrettichperoxidase versetzt, woraufhin sich je nach Menge des gebildeten Wasserstoffperoxids Methylenblau bildet. Die Menge an gebildetem Methylenblau wird photometrisch bestimmt. Alternativ zu diesem Verfahren kann das gebildete Wasserstoffperoxid auch amperometrisch bestimmt werden (siehe z.B. Hibi T. and Senda M., Biosci Biotechnol Biochem. (2000), 64:1963-1966). Da die Stabilität von Wasserstoffperoxid in einer Lösung u.a. von der Temperatur und der Anwesenheit anderer Verbindungen (z.B. fördert die Anwesenheit von Metallionen die katalytische Zersetzung des Wasserstoffperoxids, indem das Wasserstoffperoxid über radikalische Zwischenstufen die Metallionen oxidiert) beeinflusst wird, eignet sich die Bestimmung der DAO-Aktivität mittels der Wasserstoffperoxidbildungsrate nur bedingt. In Abwesenheit oxidierbarer Substanzen zersetzt sich Wasserstoffperoxid insbesondere bei höheren Temperaturen und bei Alkalität autokatalytisch, wobei der Aktivsauerstoff sich zu molekularem Sauerstoff umsetzt.

Es ist somit Aufgabe der vorliegenden Erfindung ein Verfahren zur Bestimmung der DAO-Aktivität in einer Probe zur Verfügung zu stellen, welches die eingangs beschriebenen Nachteile des Standes der Technik überwindet. Vor allem die Verwendung von radioaktiv markierten Substraten (wie z.B. Histamin, Putrescin) und die Messung von gebildetem Wasserstoffperoxid, welche in einem nicht befriedigenden Ausmaß erfolgt, soll dabei vermieden werden. Da der Einsatz von radioaktivem Material nur in Laboratorien mit spezieller Ausrüstung möglich ist und ferner die Durchführung einer Flüssigextraktion von zyklisiertem Putrescin oder Kadaverin sehr arbeitsintensiv ist, eignen sich Verfahren, in denen radioaktive Substanzen benötigt werden, bzw. Verfahren, die auf die Flüssigextraktion angewiesen sind, nicht besonders gut für den Routinebetrieb beispielsweise in einem Krankenhaus. Ferner ist die Entsorgung von radioaktiven Materialien und organischen Lösungsmitteln nicht unproblematisch und zum Teil kostenintensiv.

Daher betrifft die vorliegende Erfindung ein Verfahren zur Bestimmung der Aktivität von Diaminooxidase (DAO; EC 1.4.3.6) in einer Probe umfassend die Schritte:
- Bereitstellen einer vorgegebenen Menge eines Diamins, vorzugsweise in einer wässrigen Lösung oder lyophilisiert,
- Vermischen und Inkubieren des Diamins mit der Probe für einen festgelegten Zeitraum, unter Bedingungen, bei welchen das Diamin durch eine gegebenenfalls in der Probe vorhandene DAO umgesetzt werden kann,
- Derivatisieren des noch vorhandenen Diamins,
- Bestimmen der Menge an derivatisiertem Diamin und
- Vergleichen der vorgegebenen Menge an Diamin mit der Menge an derivatisiertem Diamin und Bestimmen der Aktivität von gegebenenfalls vorhandener Diaminooxidase.

Die Probe, die eine zu bestimmende Menge DAO enthält oder in Verdacht steht DAO zu enthalten, kann Blut (Serum oder Plasma) von Menschen oder Tieren, Gewebe oder Gewebshomogenaten humanen oder tierischen Ursprungs, z.B. mittels Biopsie gewonnene Darmepithelzellen, Cerebrospinalflüssigkeit, aus einer Körperflüssigkeit, einer Gewebeprobe, einer in vitro Zellkultur (sowohl einer bakteriellen, pflanzlichen, tierischen, menschlichen Zellkultur als auch einer Pilzkultur) oder einer sonstigen Quelle gewonnen werden. Dabei können die Proben entweder direkt oder nach einem oder mehreren Probenaufarbeitungsschritten eingesetzt werden. Diese Probenaufarbeitungsschritte hängen z.B. davon ab, wie die DAO in den isolierten Proben zur Verfügung gestellt wird. Befindet sich die DAO beispielsweise in Zellen von Gewebeproben oder Zellkulturen, müssen diese vor der Aktivitätsbestimmung unter Beibehaltung der enzymatischen Aktivität geöffnet werden (z.B. durch Ultraschallbehandlung, French Press, Lysereagentien, wie Detergentien und Enzyme, oder Homogenisieren). Ferner können der zu untersuchenden Probe auch Reagentien, Salze und Puffer zugesetzt werden, die zur Stabilisierung der DAO beitragen. Probenmatrix, die die Aktivitätsbestimmung der DAO stören bzw. derart beeinflussen, dass keine signifikante Aussage über die in der Probe tatsächlich enthaltenen DAO-Menge zulassen, sollten ebenfalls von der Probe entfernt werden. Bekanntermaßen erfolgt der Abbau des Histamins vorzugsweise durch DAO im Darmlumen, und in den Darmepithelzellen. Ein Vergleich der Aktivität von DAO in Plasma und Darmepithelzellen zeigt die bislang postulierte Korrelation. Weiters wird festgestellt, dass die Aktivität der DAO in den Darmepithelzellen um ein Vielfaches (etwa einen Faktor 100) höher als im Blut ist.

Die Probe wird mit der vorgegebenen Menge an Diamin, welches sich vorzugsweise in einer wässrigen Lösung gelöst befindet, in Kontakt gebracht und vermischt. Selbstverständlich ist es durchaus möglich eine gegebene Menge an Diamin einer definierten Menge Probe direkt (z.B. als Kristalle) hinzuzugeben. Dabei wird eine Menge an Diamin eingesetzt, die nicht zu einer signifikanten Inhibierung von DAO führt (z.B. wird DAO durch die Zugabe von mindestens 100µM Histamin zumindest teilweise inhibiert, bei einer Histamin-Konzentration von ca. 500 µM wird DAO um ca. 60% und somit signifikant inhibiert). Nach Inkubation dieses Gemisches für einen festgelegten Zeitraum, währenddessen das in der wässrigen Lösung befindliche Diamin bei Anwesenheit von DAO je nach dessen Menge abgebaut wird, wird das im Gemisch verbleibende Diamin zu 95%, vorzugsweise zu 99%, noch mehr bevorzugt zu 99,5%, insbesondere zu 100%, derivatisiert. Die Inkubationsbedingungen müssen dabei derart gewählt werden, dass die gegebenenfalls vorhandene DAO in der Probe in der Lage ist das vorhandene Diamin umzusetzen. DAO ist ein Enzym, welches Diamin-Substrate, wie z.B. Histamin, mit einer derartigen Reaktionsgeschwindigkeit umsetzt, so dass die Inkubationsdauer vorzugsweise zumindest 30 Minuten beträgt.

Zur Steigerung der Spezifität und Sensitivität kann in einer speziellen Konfiguration des Testsystems die DAO mittels eines spezifischen Antikörpers, der an einer Festphase gebunden ist, selektiv aus der Probe angereichert werden. Die Umsetzung mit Histamin oder anderen biogenen Aminen erfolgt dann ohne störende Einflüssen der Probenmatrix in einem speziellen, die DAO-Aktivität unterstützendem Puffersystem. Dieser Puffer ist vorzugsweise 10 bis 200mM, noch mehr bevorzugt 20 bis 100mM, insbesondere 50mM Tris pH = 8,5.

Nach der Derivatisierung des Diamins wird dessen Menge im Gemisch bestimmt. Die gemessene Menge an derivatisiertem Diamin wird anschließend mit der ursprünglich in der wässrigen Lösung befindlichen Diaminmenge verglichen. Die Differenz zwischen der ursprünglich eingesetzten Menge Diamin und der nach der Derivatisierung gemessenen derivatisierten Diamin-Menge gibt die Absolutmenge an abgebauten Diamin wieder. Die innerhalb eines definierten Zeitraumes abgebaute Menge an Diamin gibt die Enzymaktivität in der Probe wieder, die vorzugsweise mit Einheiten/ml (E/ml; U/ml) wiedergegeben wird.

Da - wie bereits eingangs erwähnt DAO in Säugetieren und Menschen eine wichtige Rolle spielt, kann das erfindungsgemäße Verfahren auch dazu verwendet werden, Histaminunverträglichkeit aufgrund eines DAO-Mangels oder einer reduzierten DAO-Aktivität zu diagnostizieren. Ferner eignet sich das erfindungsgemäße Verfahren auch zur Kontrolle einer Therapie bei Histamin-Intoleranz. Gesunde Individuen weisen normalerweise eine DAO-Aktivität von >10 U/ml auf (siehe z.B. Mayer I et al. (2005) Allergologie 28:1-8). Mit dem erfindungsgemäßen Verfahren ist es möglich, Aktivitäten bis unter 3 U/ml zu messen. Dadurch kann Histamin-Intoleranz bei Patienten diagnostiziert werden und/oder der Therapieverlauf kontrolliert werden.

Aufgrund der Tatsache, dass das Diamin im Zuge der Derivatisierung der DAO nicht mehr zugänglich ist bzw. dieser entzogen wird ist es nicht nötig einen DAO-Inhibitor wie unten erwähnt zur Reaktionslösung zu geben. Dennoch kann ein Inhibitor vorzugsweise dazugegeben werden. Die Derivatisierung des Diamins erfüllt somit mehrere Funktionen. Einerseits unterbindet die Derivatiserung; dass weiteres Diamin nach einem definierten Zeitraum weiter mit der DAO reagieren kann. Andererseits ermöglicht eine Derivatisierung die Detektion von vorhandenem Diamin in der Probe, denn freies Diamin ist einer Quantifizierung mittels Bindungspartnern, wie Antikörpern, nicht zugänglich.

Durch zusätzliche Zugabe einer definierten Menge an DAO bekannter Aktivität kann das Inhibitionspotential einer Probe bestimmt werden. Damit ist es möglich festzustellen, ob in der zu untersuchenden Probe ein DAO-Mangel vorliegt oder jedoch Substanzen anwesend sind, welche eine physiologisch relevante Wirkung der DAO verhindern. Derartige Substanzen können entweder hohe Mengen an anderen biogenen Aminen, wie z.B. Putrescin, Kadaverin oder Spermin sein, welche von DAO bevorzugt gegenüber Histamin abgebaut werden, oder über Arzneimittel dem Körper zugeführt wurden (bekannte DAO-Hemmer sind z.B. Acetylcystein, Ambroxol, Aminophyllin, Amitriptylin, Chloroquin, Clavulansäure, Isoniazid, Metamizol, Metodopramid und Propafenon). Durch diese Bevorzugung wird Histamin deutlich langsamer oder gar nicht mehr abgebaut und bewirkt die in der Einleitung beschriebenen Symptome.

In weiterer Folge kann diese Konfiguration dazu verwendet werden, "Inhibitor-Antagonisten" zu testen und deren Wirkung zu quantifizieren.

Erfindungsgemäß kann das Diamin mit einer Reihe von Substanzen derivatisiert werden, wobei das derivatisierte Diamin direkt (z.B. über eine Farbreaktion, durch Fluoreszenz) oder indirekt (z.B. mit Hilfe von Antikörpern oder Fragmenten davon, Derivatspezifischen Bindungspartnern) quantitativ und/oder qualitativ bestimmt werden kann. Dabei können vorzugsweise fluoreszierende Gruppen am Diamin substituiert sein, die auch bei chromatographischen Detektionssystemen Anwendung finden. Derartige Gruppen können sein: O-Phthalaldehyde (OPA) (Oguri S et al. (1999) J Chromatogr B Biomed Sci Appl 736: 263-71; Previati M et al. (2002) J Chromatogr B Analyt Technol Biomed Life Sci 780: 331-9), 9-fluorenylmethylchloroformate (Aygun O et al. (1999) J Agric Food Chem 47: 1961-4), Dansyl-Gruppen (Bolygo E et al. (2000) J AOAC Int 83: 543-8), Allophycocyanine (XL 665) (Claret EJ et al. (2003) Comb Chem High Throughput Screen 6: 789-94), Pyrene (Yoshida H et al. (2004) Anal Sci 20: 557-9) (z.B. 4-1-pryrene butyric acid N-hydroxysuccinimide ester = PSE (Yoshitake T et al. (2003) Biomed Chromatogr 17: 509-16)), 7-fluoro-4-nitrobenzoxadiazole (NBD-F) (Song Y et al. (2004) Rapid Commun Mass Spectrom 18: 2818-22), 4-(N,N-dimethylaminosulfonyl)-7-fluoro-2,1,3-benzoxadiazole (DBD-F) (Toyooka T et al. (2004) Anal Biochem 333: 236-45), Naphthalene-2,3-dicarboxaldehyde (NDA) (Zhang LY and Sun MX (2004) J Chromatogr A 1040: 133-40). Selbstverständlich können diese Substituenten auch mittels Antikörperbindung am derivatisierten Diamin bestimmt werden. Succinyl-Glycinamid wird zur Derivatisierung von Diamin in Radioimmunoassays verwendet (Morel A et al. (1990) Mol Immunol 27 (10): 995-1000).

Ein weiterer Aspekt der vorliegenden Erfindung betrifft ein Verfahren zur Identifizierung von Diaminooxidase-Inhibitoren und zur Bestimmung der Inhibitionskapazität dieser Inhibitoren umfassend die Schritte:
- Bereitstellen einer vorgegebenen Menge eines Diamins, vorzugsweise in einer wässrigen Lösung oder lyophilisiert, und von Diaminooxidase
- Vermischen und Inkubieren des Diamins und der Diaminooxidase mit einer Verbindung, die die Aktivität der Diaminooxidase potentiell inhibiert, für einen festgelegten Zeitraum
- Derivatisieren des noch vorhandenen Diamins,
- Bestimmen der Menge an derivatisiertem Diamin
- Vergleichen der vorgegebenen Menge an Diamin mit de Menge an derivatisiertem Diamin und Bestimmen der Aktivität von Diaminooxidase und
- gegebenenfalls Identifizieren von Diaminooxidase-Inhibitoren durch Vergleichen der Diaminooxidase-Aktivitäten mit und ohne Zugabe des Inhibitors, wobei ein Diaminooxidase-Inhibitor als solcher identifiziert wird, wenn die Aktivität von Diaminooxidase durch den Inhibitor um mindestens 20% gesenkt wird.

Das erfindungsgemäße Verfahren kann auch dazu verwendet werden, Inhibitoren der Diaminooxidase zu identifizieren bzw deren Inhibitionskapazität zu bestimmen. Erfindungsgemäß wird als Inhibitor eine Verbindung oder ein Gemisch von Verbindungen verstanden, die (das) in der Lage ist, die Aktivität der Diaminooxidase um mindestens 20%, vorzugsweise mindestens 40%, noch mehr bevorzugt mindestens 50% zu senken. Als Referenz für die DAO-Aktivität dient dabei eine Messung, die unter denselben Bedingungen durchgeführt wird wie die Messung in Anwesenheit des Inhibitors, wobei die DAO desselben Ursprungs ist.

Gemäß einer bevorzugten Ausführungsform ist das Diamin ausgewählt aus der Gruppe bestehend aus Histamin, Putrescin, Spermidin und Kadaverin.

Als besonders geeignet zur Verwendung im erfindungsgemäßen Verfahren sind Substrate anzusehen, die eine hohe Affinität zur DAO und gute Umsetzraten mit DAO zeigen (siehe z.B. Elmore BO et al. (2002) J Biol Inorg Chem 7:565-579). Daher eignen sich Histamin, Putrescin, Spermidin und Kadaverin besonders gut, um die DAO-Aktivität in einer Probe zu bestimmen. Ferner können diese Diamine in einfacher Weise mit bekannten Verfahren derivatisiert werden.

Die wässrige Lösung umfasst vorzugsweise eine Tris (Tris(hydroxymethyl)-aminomethan)-Pufferlösung mit einem pH-Wert von 7 bis 9,5, vorzugsweise von 7,5 bis 9, insbesondere von 8 bis 8,5.

Die Konzentration von Tris in der Pufferlösung kann dabei 10 mM bis 1 M, insbesondere 50 mM bis 1 M, betragen. Der Zusatz von NaCl in einer Konzentration von 10 bis 400 mM, vorzugsweise von 50 bis 300 mM, insbesondere von 200 mM, zur Pufferlösung wirkt sich besonders positiv auf die DAO-Aktivität aus.

Gemäß einer weiteren bevorzugten Ausführungsform enthält die wässrige Lösung ein Diamin in einer Menge von 5 bis 400, vorzugsweise von 10 bis 200 µM , insbesondere von 80 µM.

In diesem Konzentrationsbereich weist die DAO, die für das erfindungsgemäße Verfahren erforderliche Reaktionsgeschwindigkeit auf ohne die DAO signifikant zu inhibieren.

Das Inkubieren der wässrigen Lösung mit der Probe erfolgt vorzugsweise bei einer Temperatur von 10 bis 50°C, von 10 bis 40°C, von 15 bis 37°C, von 25 bis 40°C, von 18 bis 26°C, von 30 bis 37°C oder 38°C oder von 34 bis 38°C.

In diesem Temperaturbereich zeigte die DAO eine ausreichende Aktivität, um in dem erfindungsgemäßen Verfahren verwendet zu werden.

Vorzugsweise erfolgt das Inkubieren der wässrigen Lösung mit der Probe für 30 min bis 36 h, vorzugsweise für 1 bis 30 h, noch mehr bevorzugt für 3 bis 24 h, insbesondere für 18h.

Die erforderliche Inkubationszeit ist u.a. von der Inkubationstemperatur abhängig. Je mehr diese Temperatur der optimalen Temperatur der DAO angepasst wird, desto effizienter erfolgt die Umsetzungsreaktion und desto kürzer ist die Inkubationszeit. Bei Raumtemperatur (18-26°C), beispielsweise, kann die Inkubationszeit auch mehrere Stunden (z.B. 6 oder 12) betragen. Humane DAO aus Plasma, beispielsweise, hat einen Aktivitätssprung im Bereich zwischen 24°C und 28°C (deutlich reduzierte Aktivität wenn kühler). Daher muss die Inkubationszeit bei Temperaturen < 16°C signifikant verlängert werden.

Gemäß einer bevorzugten Ausführungsform wird die Inkubation der wässrigen Lösung mit der Probe und somit die Reaktion von Diamin mit der in der Probe enthaltenen DAO vor dem Derivatisieren mit Aminoguanidin, vorzugsweise in einer Konzentration von 1 mM bis 10 µM, insbesondere von 10 mM, gestoppt.

Um das Weiterreagieren der DAO mit dem Substrat nach der Inkubationszeit im Wesentlichen zu verhindern bzw. zu reduzieren, können erfindungsgemäß auch Inhibitoren eingesetzt werden.

Erfindungsgemäß können neben Aminoguanidin (Novotny WF et al. (1994) J Biol Chem 269:9921-9925) natürlich alle im Stand der Technik bekannten DAO-Inhibitoren eingesetzt werden: Acriflavine, Diazepam, N-Methyl-N-Formylhydrazin, b-Aminopropionitril, Dimaprit, O-Methylhydroxylamin, Agmantin, Ethanol (10%), Pargylin, Aldomet, Furosemid, Phenamil, Amilorid, Guanabenz, Phenelzin, Aminoguanidin, Guanfacin, Phenformin, Amitryptilin, Guanidin, Phenyprazin, Amodiaquin, Haloperidol, Promethiazin, Anserin, Hyamin 1622, Propranolol, Aziridinylalkylamin, Hydroxychloroquin, B1 Pyrimidin, Hydroxylamin, Quinacrin, Burimamid, Impromidin, Semicarbazid, Imidazolderivate, Thiamin, Carnosin, Iproniazid, Thioridazin, Chlorothiazid, Isocarboxazid, Tranylcypramin, Chlorpromazin, Isoniazid, Trimethoprim, Cimetidin, Metiamid, Tryptamin, Clonidin, Metronidazol, Tyramin, Cyanid, Nazlinin (Alkaloid) und Nt-Methylhistamin.

Gemäß einer bevorzugten Ausführungsform der vorliegenden Erfindung umfasst der Verfahrensschritt des Derivatisierens Acylieren.

Erfindungsgemäß wird unter Acylierung die Bindung einer Acylgruppe (R) einer organischen Säure mit der Formel (R-COOH) bzw. dessen Radikal (R-CO·)an die freie Aminogruppe des Histamins verstanden. Derartige Verfahren sind dem Fachmann durchwegs bekannt (Agrawal VK et al. (2001) Bioorg. Med. Chem. 9:2787-2792). Eine spezielle Art der Acylierung ist z.B. die Acetylierung, bei der die organische Säure Essigsäure ist.

Es hat sich erfindungsgemäß herausgestellt, dass sich besonders Verfahren zum Derivatisieren von Diaminen eignen, die mindestens einen Acylierungsschritt umfassen. Ferner ist es insbesondere möglich acylierte Diamine in einfacher und reproduzierbarer Weise acylierte Diamine zu detektieren und gegebenenfalls dessen Konzentration in einer Probe zu bestimmen. So können beispielsweise Antikörper, die spezifisch gegen acylierte Diamine gerichtet sind, hergestellt und in einem spezifischen Nachweisverfahren eingesetzt werden (siehe z.B. Campbell AM et al. (1993) Allergy 48:125-9).

Diamin wird vorzugsweise mit NHS-Biotin acyliert.

Erfindungsgemäß ist es aber durchaus möglich Diamin mit einer Reihe von weiteren Substanzen zu acylieren (siehe z.B. Morel AM und Delaage MA (1989) J Allergy Clin Immunol. 82:646-654).

Gemäß einer bevorzugten Ausführungsform wird die Menge an derivatisiertem Diamin mittels Chromatographie, insbesondere mit Hoch-Leistungs-Flüssig-Chromatographie (HPLC), Elektrophorese, insbesondere Kapillarelektrophorese (CE), oder Immunoassay, insbesondere Enzymimmunoassay (ELISA; Enzyme-linked Immunosorbent Assay) oder Radioimmunoassay (RIA), bestimmt.

Derivatisiertes Diamin kann mittels verschiedener Methoden quantitativ bestimmt werden, wobei sich insbesondere chromatographische, elektrophoretische und immunchemische verfahren als besonders geeignet erwiesen haben. Die quantitative Bestimmung von Molekülen, insbesondere von acyliertem Diamin, kann mit allen hierin angeführten Methoden durchgeführt werden. Alle diese Verfahren sind dem Fachmann hinreichend bekannt und können ohne weiteres und mit wenig und somit zumutbarem Aufwand auf die Quantifizierung von einer Vielzahl von mit verschiedenen Substanzen derivatisierten Diaminen adaptiert werden (siehe z.B. Garcia-Villar N et al. (2005) Analyst 130:1286-1290; Yoshitake T et al. (2003) J Neurosci Methods 127:11-17).

Besonders bevorzugt wird derivatisiertes Diamin mit immunchemischen Methoden quantifiziert, da derartige Methoden einen Einsatz in der Routine, wie z.B. in Krankenhäusern, ohne allzu großen apparativen Aufwand ermöglichen. Besonders ELISA und RIA eignen sich sehr gut für den erfindungsgemäßen Zweck. Voraussetzung für die Verwendung immunchemischer Methoden im erfindungsgemäßen Verfahren ist die Bereitstellung eines Antikörpers, der in der Lage ist derivatisiertes Diamin von nicht-derivatisiertem Diamin zu unterscheiden. Derartige Antikörper können z.B. durch Injektion von derivatisiertem Diamin in Tieren (z.B. Kaninchen, Schafe, Mäuse) oder sonstige dem Fachmann bekannte Methoden (z.B. Köhler G. und Milstein C., Nature (1975) 256:495-7) hergestellt werden. Beispielsweise wird in der US 4,818,683 die Herstellung von Antikörpern gerichtet gegen acylierten Histamin beschrieben, wobei diese Antikörper zur Bestimmung von Histamin in einer Probe herangezogen werden können.

Die immunchemischen Verfahren, insbesondere ELISA, können sowohl kompetitiv als auch einfach durchgeführt werden. Es ist beispielsweise möglich, einen festen Träger, vorzugsweise die Wells von Mikrotiterplatten (mit 96, 384 oder mehr Wells), mit derivatiserten Diamin zu beschichten. Die so beschichteten Wells können anschließend mit einem Gemisch aus derivatisiertem Diamin, welches aus der Umsetzungsreaktion von Diamin mit der Probe, umfassend gegebenenfalls DAO, und anschließender Derivatisierung hergestellt wurde, und einem Diaminderivat-spezifischen Antikörper in Kontakt gebracht werden. Das im Well gebundene Diaminderivat konkurriert mit dem aus der Umsetzungsreaktion gewonnenen derivatisierten Diamin um die Diaminderivat-spezifischen Antikörper in der Lösung. Während der Inkubation im Well stellt sich ein Gleichgewicht ein, wobei die Menge an Antikörpern, welche am Diaminderivat des Wells gebunden ist, größer ist je mehr Diamin mit DAO umgesetzt wurde. Die im Well am Diaminderivat gebundenen Antikörper können nach einem Waschschritt entweder mit einem weiteren, gegen den Diaminderivat-spezifischen Antikörper gerichteten Antikörper, der vorzugsweise Enzym-markiert (z.B. mit Meerrettich-Peroxidase) ist, durch ein Farbreaktion quantifiziert werden. Anhand einer parallel durchgeführten Bestimmung einer Standardreihe kann auf die abgebaute Menge an Diamin geschlossen werden. Alternativ dazu könnte der Diaminderivat-spezifische Antikörper selbst mit z.B. einem Enzym (wie Meerrettich-Peroxidase, alkalische Phosphatase) markiert sein. Alternativ ist es möglich, einen gegen das Diaminderivat gerichteten Antikörper in den Wells zu immobilisieren, wobei dieser Antikörper sowohl markiertes (z.B. mit Enzymen) als auch unmarkiertes Diaminderivat zu binden vermag. Bei der Bestimmung von nicht von der Probe umgesetztem Diamin konkurriert dieses mit zugesetztem markierten Diaminderivat um die freien Antikörperbindungsstellen. Je mehr unmarkiertes Diaminderivat vorhanden ist desto weniger markiertes Diaminderivat kann über den Bindungsantikörper an das Well binden. Die Bestimmung von markiertem Antikörper bzw. Diaminderivat erfolgt mit dem Fachmann bekannten Verfahren. Weiters ist es möglich, wenn beispielsweise NHS-Biotin zum Derivatisieren des Diamins verwendet wird, einen festen Träger mit Streptavidin zu versehen. Die an dem festen Träger (z.B. die Wells einer Mikrotiterplatte) immobilisierten Streptavidin-Moleküle können anschließend spezifisch das Biotin des derivatisierten Diamins binden. Von DAO umgesetztes Diamin kann somit nicht am festen Träger gebunden werden. Das am festen Träger über Streptavidin gebundene derivatisierte Diamin kann anschließend mit einem Antikörper, der spezifisch gegen derivatisiertes Diamin gerichtet ist, direkt oder indirekt detektiert und gegebenenfalls quantifiziert werden. Selbstverständlich kann anstelle von Streptavidin ein anderer Bindungspartner verwendet werden, vorausgesetzt dieser ist in der Lage derivatisiertes Diamin zu binden und dennoch die Zugänglichkeit von für das derivatisierte Diamin spezifischen Antikörpern zu ermöglichen.

Im Prinzip können erfindungsgemäß alle in der Literatur beschriebenen Verfahren zur Detektion von derivatisierten Diaminen, insbesondere von Diaminderivat, herangezogen werden (siehe z.B. Claret EJ et al. Comb Chem High Throughput Screen (2003) 6:789-94).

Bei der Derivatisierung ist darauf zu achten, dass auch andere Substanzen in der Probe, die freie Aminogruppen aufweisen, acyliert werden können. Daher sollte einerseits jedenfalls zunächst ein Leerwert bestimmt werden und andererseits das Verfahren zur Bestimmung des acylierten Diamins spezifisch sein, bei dem z.B. ein Bindungspartner, wie ein Antikörper, eingesetzt wird, der im Wesentlichen lediglich das acylierte Diamin zu erkennen vermag.

Diaminooxidase kann teilweise auch von einem ihrer Reaktionsprodukte, dem Wasserstoffperoxid, gehemmt werden (Pietrageli et al. (2004) Eur. J. Biochem. 271, 146-152). Um diese Hemmung zu verhindern bzw. zu reduzieren, wird - gemäß einer bevorzugten Ausführungsform der vorliegenden Erfindung - zur wässrigen Lösung und/oder zur Probe Peroxidase (EC 1.11.1), vorzugsweise Katalase (EC 1.11.1.6), zugesetzt.

Gemäß einer bevorzugten Ausführungsform wird die Peroxidase bzw. Katalase in einer Menge von 0,1 bis 10, vorzugsweise von 0,2 bis 5, noch mehr bevorzugt von 0,5 bis 2, insbesondere von 1, Einheiten pro 100µl Probe zugesetzt.

Ein weiterer Aspekt der vorliegenden Erfindung betrifft ein Set zur Bestimmung der Aktivität von Diaminooxidase (DAO; EC 1.4.3.6) in einer Probe umfassend:
- Diamin-Derivatisierungsreagens,
- Mittel zur Bestimmung von derivatisiertem Diamin,
- gegebenenfalls Peroxidase, vorzugsweise Katalase (vorzugsweise in lyophilisierter Form), stabilisiertes DAO-Präparat mit einer definierten Menge an DAO
- gegebenenfalls DAO-Puffer und
- gegebenenfalls Diamin als Substrat.

Zur Bestimmung der Aktivität von Diaminooxidase in einer Probe, vorzugsweise nach dem erfindungsgemäßen Verfahren, eignet sich insbesondere ein Set wie hierin definiert. Dabei umfasst das Derivatisierungsreagens im Wesentlichen die für die Derivatisierung eines Diamins erforderlichen Chemikalien, Puffer und dergleichen. Als wichtigster und gegebenenfalls einziger Bestandteil des Derivatisierungsreagens ist oder sind jene Reagentien anzusehen, die für die Derivatisierung des Diamins verantwortlich sind (z.B. Acylgruppendonor). Ferner kann das Set Mittel zur Bestimmung von derivatisiertem Diamin enthalten. Diese Mittel umfassen vorzugsweise Antikörper gerichtet gegen derivatisiertes Diamin, insbesondere polyklonale Antikörper, und gegebenenfalls sekundäre Antikörper, wobei mindestens einer dieser Antikörper vorzugsweise enzymatisch oder chemisch markiert ist. Die dabei vorgesehenen Antikörper können je nach Verwendung von verschiedensten Quellen stammen (z.B. von einer Zellkultur, von einem Tier; wie Schaf, Kaninchen oder Maus) und markiert (z.B. mit einem Enzym, mit Biotin, mit Streptavidin, mit einem Radionuklid) oder unmarkiert sein.

Zur Durchführung einer Kontrollreaktion, bei der eine definierte Menge an DAO eingesetzt werden kann, kann das erfindungsgemäße Set auch ein stabilisiertes DAO-Präparat enthalten. Dieses DAO-Präparat kann beispielsweise lyophilisiertes oder kältestabilisiertes DAO (geeignet zum Lagern bei Temperaturen zwischen -30°C und -10°C oder zwischen 1°C und 6°C) enthalten.

Ferner kann das erfindungsgemäße Set auch einen DAO-Puffer umfassen, welcher sich insbesondere zur Aktivitätsbestimmung von DAO eignet. Der DAO-Puffer kann im Set in konzentrierter Form (z.B. als 5-, 10-, 20- oder 50-fach Konzentrat) vorgesehen sein.

Das erfindungsgemäße Set kann auch acyliertes Diamin enthalten, welches als Standard bei der Bestimmung der Enzymaktivität, insbesondere bei der Bestimmung der Menge an derivatisiertem Diamin, herangezogen werden kann.

Die vorliegende Erfindung wird weiters durch die folgenden Figuren und Beispiele dargelegt, ohne jedoch auf diese beschränkt zu sein.

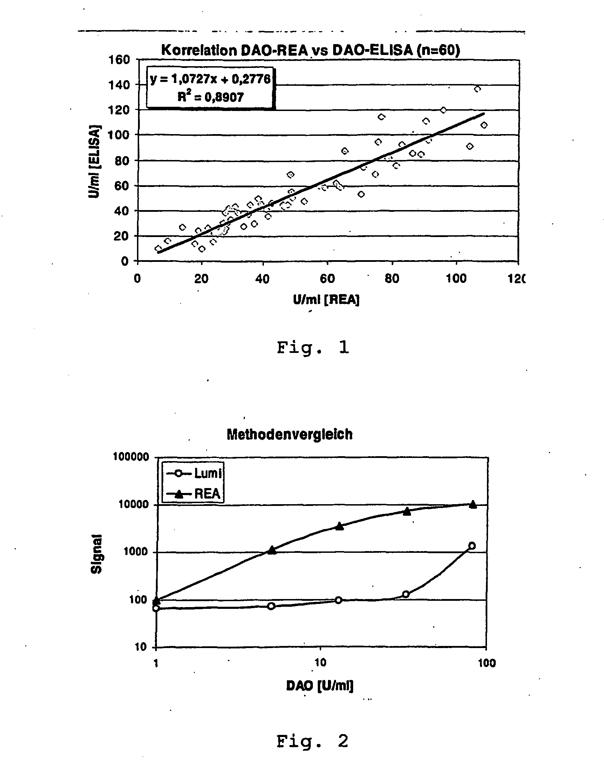

Fig. 1 zeigt die Korrelation der Messergebnisse der Bestimmung der Enzymaktivität in einer Probe mit einer definierten Menge an DAO zwischen dem erfindungsgemäßen Verfahren und einem Verfahren unter Verwendung von radioaktiv markiertem Diamin (AT 411.688).
Fig. 2 zeigt einen Sensitivitätsvergleich zwischen einem Verfahren zur Bestimmung der DAO-Aktivität in einer Probe unter Verwendung von radioaktiv-markiertem Diamin und einem Verfahren zur Detektion von beim Diamin-Abbau durch DAO gebildeten Wasserstoffperoxid.
Fig. 3 zeigt die relative Enzymaktivität einer definierten Menge DAO in einer Probe in Abhängigkeit der eingesetzten Histaminmenge.
Fig. 4 zeigt den Vergleich einer DAO-Standardkurve mit und ohne Zugabe von Katalase.

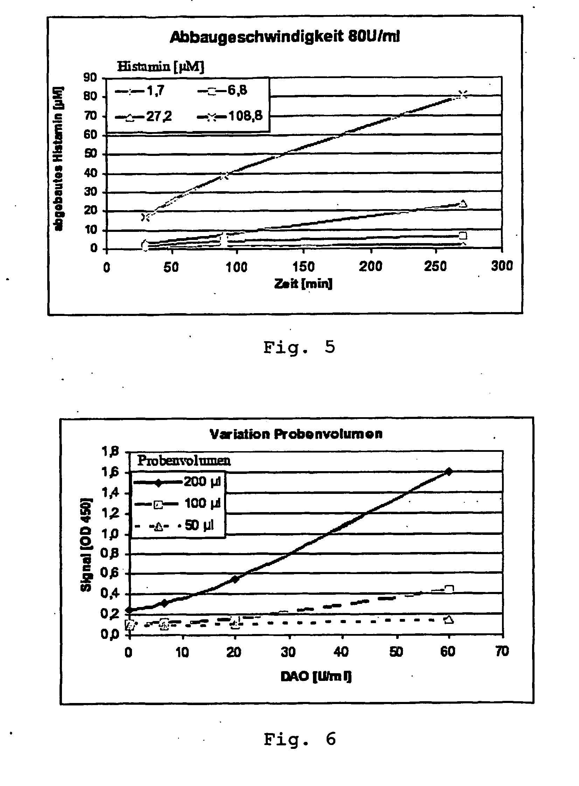

Fig. 5 zeigt die Abbaugeschwindigkeit von Histamin bei einer DAO-Aktivität von 80 U/ml. Der Umsatz von Histamin ist gemäß der Michaelis-Menten-Charakteristik des Enzyms von der Konzentration des eingesetzten Histamins abhängig. Für eine DAO-Aktivität von 80 U/ml ist der Umsatzverlauf in dieser Figur angegeben.
Fig. 6 zeigt die Abhängigkeit der Empfindlichkeit des erfindungsgemäßen Verfahrens vom eingesetzten Probenvolumen.
Fig. 7 zeigt die Aktivität von aus einem Lyophilisat hergestellter DAO. Um optimale Stabilität des Testsystems zu gewährleisten, wird DAO für die Standardkurve in lyophilisierter Form hergestellt. Die gesamte Aktivität ist bereits 15 min nach Auflösung verfügbar und bleibt auch nach drei Tagen bei Raumtemperatur stabil.
Fig. 8 zeigt die Korrelation zwischen der DAO-Aktivität im Plasma und im Darmepithel.

### BEISPIELE:

### Beispiel 1:

Frisch abgenommene Proben (Plasma oder Serum) wurden nach der Abnahme bei 4°C aufbewahrt. Die Proben können bei 4°C bis zu 2 Tage lang, für längere Zeit bei -20°C aufbewahrt werden.
Tag 1:
   - Standards wurden in 1,0 ml aqua deion für 15 min bei Raumtemperatur aufgelöst und gut gemischt (Vortex)
   - Am mitgelieferten Ansatzprotokoll wurden die Positionen für Standards und Proben markiert
   - Je 200 µl von Standard und Probe wurden in die jeweiligen Wells der Incubation Plate (Vorbereitung: 0,6 µl Histamin (1 mg/ml in 50 mM Tris p H8), 0,2 µl Katalase, Kristallsuspension in Wasser, 0,12 g Saccharose, 120 µl 1M Tris, 2,3 ml RO-Wasser mischen und 25 µl in jedes Well pipettieren) pipettiert
   - Streifen wurden abgedeckt und über Nacht bei Raumtemperatur (18-26°C) inkubiert. Nicht verwendete Streifen wurden im Alubeutel mit Trockenmittelbeutel bei 4°C gelagert
Tag 2:
   - 10 x Waschpufferkonzentrat wurde mit aqua deion. 1 + 9 verdünnt (z.B. 60 ml Waschpufferkonzentrat plus 540 ml aqua deion.) (Endkonzentration: 100 mM Tris-HCl pH = 9,0)
   - Acylierungsreagens (Biotin-NHS lyophilisiert; nach Bedarf mehrere Fläschchen) wurde mit Acylierungs-Diluent (DMSO (Dimethylsulfoxid) + DMFO (Dimethylformamid) 1:1) gelöst (1 ml pro Fläschchen), evtl. gepoolt, gut gemischt. Das gelöste Reagens sollte innerhalb von 30 min verwendet werden.
   - 50 µl des gelösten Acylierungsreagens wurde in jedes Well der Incubation Plate mit Probe pipettiert (z.B. mit Multipette). Nicht verwendete Streifen wurden im Alubeutel mit Trockenmittelbeutel bei 4°C gelagert
   - Streifen wurden 30 min am Schüttler bei 37°C inkubiert
   - 150 µl Assaypuffer (50 mM Tris pH 8, 100 mM NaCl, 2% PEG) wurden in die benötigten Wells der Enhancer Plate (Vorbereitung: 24 ml Antikörperpuffer (50 mM Tris pH 8, 100 mM NaCl, 2% PEG, 5% Saccharose) vorlegen, 19 µl Serum anti Acyl-Histamin (z.B. Ziegenserum) zusetzen und mischen und in jedes Well pipettieren) pipettiert
   - 50 µl Probe aus der Incubation Plate wurden in die entsprechenden Wells der Enhancer Plate transferiert (Mehrkanalpipette). Nicht verwendete Streifen werden im Alubeutel mit Trockenmittelbeutel bei 4°C gelagert
   - Streifen wurden abgedeckt und 45 min bei 37°C am Schüttler inkubiert
   - 150 µl Reaktionsgemisch aus der Enhancer Plate wurden in die entsprechenden Wells der ELISA-Plate transferiert (Mehrkanalpipette). Nicht verwendete Streifen wurden im Alubeutel mit Trockenmittelbeutel bei 4°C gelagert
   - Streifen wurden abgedeckt und 90 min bei 37°C,am Schüttler inkubiert
   - der Inhalt der Wells wurde verworfen und die Wells 4-mal mit 350 µl verdünntem Waschpuffer gewaschen
   - 100 µl Konjugat (z.B. Kaninchen Anti-Ziegen - HRPO 1:5000 on PO-Stabilizer; Hersteller e-bioscience) wurde in alle Wells pipettiert
   - Streifen wurden abgedeckt und 30 min bei 37°C am Schüttler inkubiert
   - der Inhalt der Wells wurde verworfen und die Wells 4 mal mit 350 µl verdünntem Waschpuffer gewaschen
   - 100 µl Substrat (TMB (Tetramethylbenzidin) gebrauchsfertig von Sigma) wurde in alle Wells pipettiert und 15 min bei RT (18-26°C) inkubiert
   - 50 µl Stop-Lösung (1M HCl) wurde in alle Wells pipettiert
   - die Optische Dichte wurde in einem ELISA-Reader bei 450 nm (Referenz: 620 nm) bestimmt

### Beispiel 2:

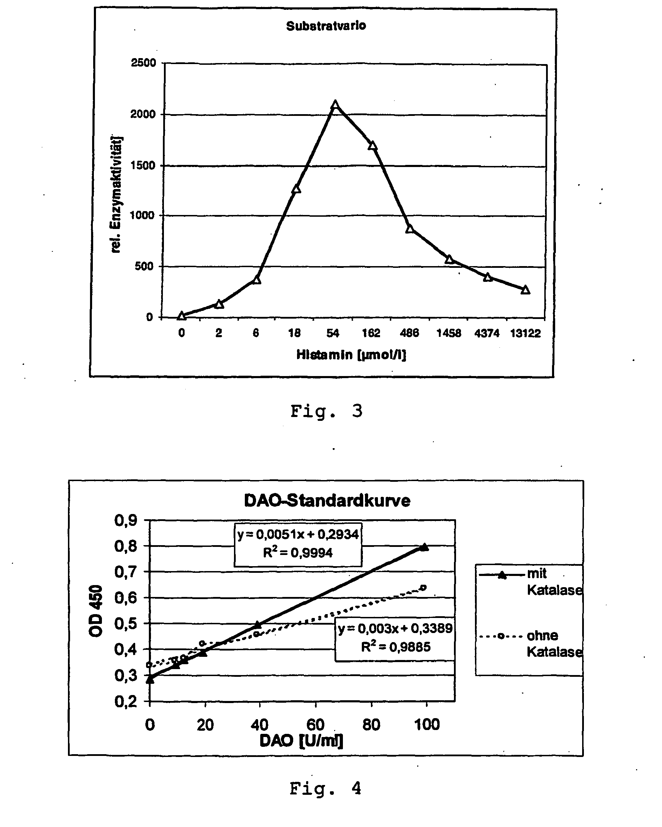

Um die Hemmung von DAO durch Wasserstoffperoxid hintanzuhalten, wurde der Probe Katalase zugesetzt. Das brachte eine signifikante Verbesserung der Steigung und eine wesentlich verbesserte Linearität der Standardkurve (siehe Fig. 4).

Die Katalase wurde unmittelbar vor der Reaktion von DAO mit Histamin der Probe zugesetzt, die Aktivität beträgt 2 Enzymeinheiten Katalase pro 200 µl Probe. Im Speziellen wurde die Katalase lyophilisiert gemeinsam mit Histamin in der Incubation Plate vorgelegt.

### Beispiel 3:

Bekanntermaßen erfolgt der Abbau des Histamins vorzugsweise durch DAO im Darmlumen und in den Darmepithelzellen. Ein Vergleich der Aktivität von DAO in Plasma und Darmepithelzellen zeigt die bislang postulierte Korrelation. Weiters wird festgestellt, dass die Aktivität der DAO in den Darmepithelzellen um ein Vielfaches (etwa einen Faktor 100) höher als im Blut ist.

10mg Biopsiematerial wurden in 50mM Trispuffer (pH=8,5+100mM NaCl) homogenisiert. Das Homogenat wurde im Puffer 1:300 verdünnt, um in den messbaren Bereich zu kommen. Diese Verdünnung wurde im Test analog zur Plasmaprobe gemessen.

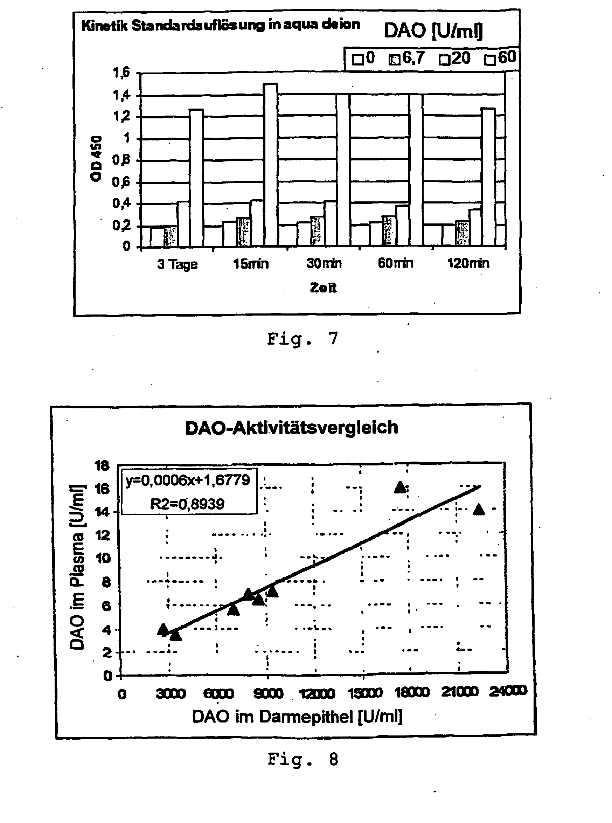

In Fig. 8 werden die Ergebnisse des DAO-Aktivitätsvergleichs graphisch dargestellt.

### Beispiel 4:

In Beispiel 4 werden dieselben Proben wie in Beispiel 1 verwendet. Beispiel 4 stellt eine weitere Möglichkeit dar, das erfindungsgemäße Verfahren durchzuführen.
Tag 1:
   1. Standards in 1 ml Assay Reagenz (Wasser mit Konservierungsmittel) auflösen; 15 min bei RT lösen lassen, gut mischen (Vortex). Nicht benötigte Standards können bis zu 3 Tage bei 4°C gelagert werden, längere Lagerung bis zum Ablaufdatum bei -20°C.
   2. Kontrolle in 1 ml Assay Reagenz auflösen; 15 min bei RT lösen lassen, gut mischen (Vortex). Nicht benötigte Kontrolle kann bis zu 3 Tage bei 4°C gelagert werden, längere Lagerung bis zum Ablaufdatum bei -20°C.
   3. An einem Ansatzprotokoll die Positionen für Standards, Kontrolle und Proben markieren.
      Entnehmen der benötigten Streifen der Inkubationsplatte aus dem Beutel (Vorbereitung: 0,6 µl Histamin (1 mg/ml in 50 mM Tris p H8), 0,2 µl Katalase, Kristallsuspension in Wasser, 0,12 g Saccharose, 120 µl 1M Tris, 2,3 ml RO-Wasser mischen und 25 µl in jedes Well pipettieren, lyophilisieren).
      Nicht verwendete Streifen im Alubeutel mit Trockenmittelbeutel bei 4°C lagern.
   4. Je 50 µl von Standard, Kontrolle und Probe in die jeweiligen Wells der Inkubationsplatte pipettieren.
   5. 50 µl Assay Reagenz in alle Wells pipettieren, leicht schütteln.
   6. Streifen abdecken und über Nacht bei 37°C inkubieren (wenn möglich schütteln).
Tag 2:
   7. 10 x Waschpufferkonzentrat mit aqua deion. 1 + 9 verdünnen (z.B. 60 ml Waschpufferkonzentrat plus 540 ml aqua deion.; Endkonzentration: 100 mM Tris-HCl pH = 9,0 + 0,05% Bridge 35).
   Das verdünnte Wasspufferkonzentrat ist bei 4°C haltbar.
   8. Antikörper (z.B. Ziege anti Histamin-Derivat) in 20 ml Assay Puffer (50 mM Tris pH = 8,05 + 100 mM NaCl) auflösen.
   9. Acylierungsreagenz (Biotin-NHS lyophilisiert; nach Bedarf mehrere Fläschchen) mit Acylierungs-Verdünnungsmittel (DMSO (Dimethylsulfoxid) + DMFO (Dimethylformamid) 1:1) lösen (1 ml pro Fläschchen), evtl. poolen, gut mischen. Das gelöste Reagenz sollte innerhalb von 30 min verwendet werden.
   10. 20 µl des gelösten Acylierungsreagenz in jedes Well der Inkubationsplatte mit Probe pipettieren.
   11. Streifen 30 min bei 37°C inkubieren (eventuell vorsichtig schütteln), NICHT abdecken!
   12. 150 µl Assay Reagenz in jedes Well pipettieren.
   13. Streifen 30 min bei 37°C inkubieren (eventuell vorsichtig schütteln), NICHT abdecken!
   14. Benötigte Streifen der ELISA-Platte (beschichtet mit derivatisiertem Diamin) aus dem Beutel entnehmen.

   Nicht verwendete Streifen im Alubeutel mit Trockenmittelbeutel bei 4°C lagern.
   15. 30 µl Probe aus der Inkubationsplatte in die entsprechenden Wells der ELISA-Platte transferieren.
   16. 150 µl der aufgelösten Antikörper-Lösung (siehe Punkt 8) in die entsprechenden Wells der ELISA-Platte pipettieren.
   17. Streifen abdecken und 45 min bei 37°C inkubieren (eventuell schütteln).
   18. Inhalt der Wells verwerfen und 4 mal mit 300 µl verdünntem Waschpuffer waschen.
   19. 100 µl Konjugat (z.B. Kaninchen anti Ziege-HRPO) in alle Wells pipettieren.
   20. Streifen abdecken und 30 min bei 37°C inkubieren (eventuell schütteln).
   21. Inhalt der Wells verwerfen und 4 mal mit 350 µl verdünntem Waschpuffer waschen.
   22. 100 µl Substrat (z.B. Tetramethylbenzidin / Wasserstoffperoxid Gemisch) in alle Wells pipettieren.
   23. 15 min bei RT (18-26°C) inkubieren.
   24. 50 µl Stoplösung (z.B. 1M HCl) in alle Wells pipettieren.
   25. Optische Dichte in einem ELISA-Reader bei 450 nm (Referenz: 620 nm) bestimmen.

## Patentansprüche

1. Verfahren zur Bestimmung der Aktivität von Diaminooxidase (DAO; EC 1.4.3.6) in einer Probe umfassend die Schritte:
- Bereitstellen einer vorgegebenen Menge eines Diamins, vorzugsweise in einer wässrigen Lösung oder lyophilisiert,
- Vermischen und Inkubieren des Diamins mit der Probe für einen festgelegten Zeitraum, unter Bedingungen, bei welchen das Diamin durch eine gegebenenfalls in der Probe vorhandene DAO umgesetzt werden kann,
- Derivatisieren des noch vorhandenen Diamins,
- Bestimmen der Menge an derivatisiertem Diamin und
- Vergleichen der vorgegebenen Menge an Diamin mit der Menge an derivatisiertem Diamin und Bestimmen der Aktivität von gegebenenfalls vorhandener Diaminooxidase.

2. Verfahren zur Identifizierung von Diaminooxidase-Inhibitoren und zur Bestimmung der Inhibitionskapazität dieser Inhibitoren umfassend die Schritte:
- Bereitstellen einer vorgegebenen Menge eines Diamins, vorzugsweise in einer wässrigen Lösung oder lyophilisiert, und von Diaminooxidase
- Vermischen und Inkubieren des Diamins und der Diaminooxidase mit einer Verbindung, die die Aktivität der Diaminooxidase potentiell inhibiert, für einen festgelegten Zeitraum
- Derivatisieren des noch vorhandenen Diamins,
- Bestimmen der Menge an derivatisiertem Diamin
- Vergleichen der vorgegebenen Menge an Diamin mit de Menge an derivatisiertem Diamin und Bestimmen der Aktivität von Diaminooxidase und
- gegebenenfalls von Diaminooxidase-Inhibitoren durch Vergleichen der Diaminooxidase-Aktivitäten mit und ohne Zugabe des Inhibitors, wobei ein Diaminooxidase-Inhibitor die Aktivität von Diaminooxidase um mindestens 20% senkt.

3. Verfahren nach Anspruch 1 oder 2, **dadurch gekennzeichnet, dass** das Diamin ausgewählt aus der Gruppe bestehend aus Histamin, Putrescin, Spermidin und Kadaverin ist.

4. Verfahren nach einem der Ansprüche 1 bis 3, **dadurch gekennzeichnet, dass** die wässrige Lösung eine Tris (Tris(hydroxymethyl)-aminomethan)-Pufferlösung mit einem pH-Wert von 7 bis 9,5, vorzugsweise von 7,5 bis 9, insbesondere von 8 bis 8,5 umfasst.

5. Verfahren nach einem der Ansprüche 1 bis 4, **dadurch gekennzeichnet, dass** die wässrige Lösung Diamin in einer Menge von 5 bis 400, vorzugsweise von 10 bis 200 µM , insbesondere von 80 µM, enthält.

6. Verfahren nach einem der Ansprüche 1 bis 5, **dadurch gekennzeichnet, dass** das Inkubieren der wässrigen Lösung mit der Probe bei einer Temperatur von 10 bis 50°C, vorzugsweise von 25 bis 40°C, noch mehr bevorzugt von 30 bis 38°C, insbesondere von 34 bis 38°C erfolgt.

7. Verfahren nach einem der Ansprüche 1 bis 6, **dadurch gekennzeichnet, dass** das Inkubieren der wässrigen Lösung mit der Probe für 30 min bis 36 h, vorzugsweise für 1 bis 30 h, noch mehr bevorzugt für 3 bis 24 h, insbesondere für 18h, erfolgt.

8. Verfahren nach einem der Ansprüche 1 bis 7, **dadurch gekennzeichnet, dass** die Inkubation der wässrigen Lösung mit der Probe vor dem Derivatisieren mit Aminoguanidin gestoppt wird.

9. Verfahren nach einem der Ansprüche 1 bis 8, **dadurch gekennzeichnet, dass** der Verfahrensschritt des Derivatisierens Acylieren umfasst.

10. Verfahren nach Anspruch 9, **dadurch gekennzeichnet, dass** Diamin mit NHS-Biotin acyliert wird.

11. Verfahren nach einem der Ansprüche 1 bis 10, **dadurch gekennzeichnet, dass** die Menge an derivatisierten Diamin mittels Chromatographie, insbesondere mit Hoch-Leistungs-Flüssig-Chromatographie (HPLC), Elektrophorese, insbesondere Kapillarelektrophorese (CE), oder Immunoassay, insbesondere Enzymimmunoassay (ELISA; Enzyme-linked Immunosorbent Assay) oder Radioimmunoassay (RIA), bestimmt wird.

12. Verfahren nach einem der Ansprüche 1 bis 11, **dadurch gekennzeichnet, dass** zur wässrigen Lösung und/oder zur Probe Peroxidase, vorzugsweise Katalase, zugesetzt wird.

13. Verfahren nach Anspruch 12, **dadurch gekennzeichnet, dass** die Peroxidase in einer Menge von 0,1 bis 10, vorzugsweise von 0,2 bis 5, noch mehr bevorzugt von 0,5 bis 2, insbesondere von 1, Einheiten Peroxidase pro 100 µl Probe zugesetzt wird.

14. Verfahren nach einem der Ansprüche 1 bis 13, **dadurch gekennzeichnet, dass** vor dem Vermischen und Inkubieren des Diamins mit der Probe, die Diaminoxidase in der Probe mittels eines spezifischen Antikörpers, der an einer Festphase gebunden ist, selektiv aus der Probe angereichert wird.

15. Verwendung eines Sets umfassend:
- Diamin-Derivatisierungsreagens,
- Mittel zur Bestimmung von derivatisiertem Diamin-,
- stabilisiertes DAO-Präparat mit einer definierten Menge an DAO,
- DAO-Puffer,
- gegebenenfalls Peroxidase, vorzugsweise Katalase, und
- gegebenenfalls Diamin als Substrat, zur Bestimmung der Aktivität von Diaminooxidase (DAO; EC 1.4.3.6) in einer Probe.

16. Verwendung nach Anspruch 15, **dadurch gekennzeichnet, dass** das Mittel zur Bestimmung von derivatisierten Diamin Antikörper gerichtet gegen derivatisierten Diamin, insbesondere polyklonale Antikörper, und gegebenenfalls sekundäre Antikörper umfasst, wobei mindestens einer dieser Antikörper vorzugsweise enzymatisch oder chemisch markiert ist.

## Claims

1. A method for determining the activity of diamine oxidase (DAO; EC 1.4.3.6) in a sample, comprising the steps of:
- providing a given amount of a diamine, preferably in an aqueous solution or lyophilized,
- mixing and incubating the diamine with the sample for a fixed period under conditions at which the diamine can be reacted by a DAO possibly present in the sample,
- derivatizing the still present diamine,
- determining the amount of derivatized diamine, and
- comparing the given amount of diamine with the amount of derivatized diamine, and determining the activity of possibly present diamine oxidase.

2. A method for identifying diamine oxidase inhibitors and for determining the inhibition capacities of said inhibitors, comprising the steps of:
- providing a given amount of a diamine, preferably in an aqueous solution or lyophilized, and of diamine oxidase,
- mixing and incubating the diamine and the diamine oxidase with a compound potentially inhibiting the activity of diamine oxidase, for a fixed period
- derivatizing the still present diamine,
- determining the amount of derivatized diamine,
- comparing the given amount of diamine with the amount of derivatized diamine, and determining the activity of diamine oxidase, and
- optionally of diamine oxidase inhibitors by comparing the diamine oxidase activities with and without the addition of an inhibitor, a diamine oxidase inhibitor lowering the activity of diamine oxidase by at least 20%.

3. A method according to claim 1 or 2, **characterized in that** the diamine is selected from the group consisting of histamine, putrescine, spermidine and cadaverine.

4. A method according to any one of claims 1 to 3, **characterized in that** the aqueous solution comprises a Tris (Tris(hydroxymethyl)-aminomethane) buffer solution having a pH of from 7 to 9.5, preferably 7.5 to 9, in particular 8 to 8.5.

5. A method according to any one of claims 1 to 4, **characterized in that** the aqueous solution contains a diamine in an amount of from 5 to 400 µM, preferably 10 to 200 µM, in particular 80 µM.

6. A method according to any one of claims 1 to 5, **characterized in that** the incubation of the aqueous solution with the sample takes place at a temperature of from 10 to 50°C, preferably 25 to 40°C, even more preferred 30 to 38°C, in particular from 34 to 38°C.

7. A method according to any one of claims 1 to 6, **characterized in that** the incubation of the aqueous solution with the sample is effected for 30 min to 36 h, preferably 1 to 30 h, even more preferred 3 to 24 h, in particular 18 h.

8. A method according to any one of claims 1 to 7, **characterized in that** the incubation of the aqueous solution with the sample is stopped before derivatizing with amino guanidine.

9. A method according to any one of claims 1 to 8, **characterized in that** the method step of derivatizing comprises acylating.

10. A method according to claims 9, **characterized in that** diamine is acylated with NHS biotin.

11. A method according to any one of claims 1 to 10, **characterized in that** the amount of derivatized diamine is determined by chromatography, in particular high-performance liquid chromatography (HPLC), electrophoresis, in particular capillary electrophoresis (CE), or immunoassay, in particular enzyme-linked immunosorbent assay (ELISA) or radioimmunoassay (RIA).

12. A method according to any one of claims 1 to 11, **characterized in that** peroxidase, preferably catalase, is admixed to the aqueous solution and/or the sample.

13. A method according to claim 12, **characterized in that** the peroxidase is admixed in an amount of from 0.1 to 10, preferably 0.2 to 5, even more preferred 0.5 to 2, in particular 1, unit(s) of peroxidase per 100 µl sample.

14. A method according to any one of claims 1 to 13, **characterized in that** prior to admixing and incubating the diamine with the sample, the diamine oxidase in the sample is selectively enriched from the sample by means of a specific antibody bound to a solid phase.

15. Use of a kit for the determination of the activity of diamine oxidase (DAO; EC 1.4.3.6) in a sample, comprising:
- a diamine derivatization reagent,
- means for determining derivatized diamine,
- a stabilized DAO preparation containing a defined amount of DAO,
- a DAO buffer,
- optionally peroxidase, preferably catalase, and
- optionally a diamine as a substrate.

16. Use of a kit according to claim 15, **characterized in that** said means for determining derivatized diamine comprises antibodies directed against derivatized diamine, in particular polyclonal antibodies, and optionally secondary antibodies, at least one of these antibodies preferably being enzymatically or chemically labelled.

## Revendications

1. Procédé pour déterminer l'activité de la diaminooxydase (DAO ; EC 1.4.3.6) dans un échantillon comprenant les étapes :
- préparation d'une quantité prédéterminée d'une diamine, de préférence dans une solution aqueuse ou lyophilisée,
- mélange et incubation de la diamine avec l'échantillon pendant une durée déterminée, dans des conditions dans lesquelles la diamine peut être convertie par une DAO éventuellement présente dans l'échantillon,
- dérivatisation de la diamine encore présente,
- détermination de la quantité de diamine dérivatisée et
- comparaison de la quantité prédéterminée de diamine avec la quantité de diamine dérivatisée et détermination de l'activité de la diaminooxydase éventuellement présente.

2. Procédé pour identifier des inhibiteurs de diaminooxydase et pour déterminer la capacité d'inhibition de ces inhibiteurs comprenant les étapes :
- préparation d'une quantité prédéterminée d'une diamine, de préférence dans une solution aqueuse ou lyophilisée, et de diamino-oxydase,
- mélange et incubation de la diamine et de la diaminooxydase avec un composé qui inhibe potentiellement l'activité de la diamino-oxydase, pendant une durée déterminée
- dérivatisation de la diamine encore présente,
- détermination de la quantité de diamine dérivatisée
- comparaison de la quantité prédéterminée de diamine avec la quantité de diamine dérivatisée et détermination de l'activité de la diamino-oxydase, et
- éventuellement d'inhibiteurs de diaminooxydase par comparaison des activités de la diaminooxydase avec et sans addition de l'inhibiteur, où un inhibiteur de diaminooxydase abaisse d'au moins 20 % l'activité de la diaminooxydase.

3. Procédé selon la revendication 1 ou 2 **caractérisé en ce que** la diamine est choisie dans le groupe consistants en l'histamine, la putrescine, la spermidine et la cadavérine.

4. Procédé selon l'une des revendications 1 à 3 **caractérisé en ce que** la solution aqueuse comprend une solution de tampon tris (tris(hydroxyméthyl)-aminométhane) ayant un pH de 7 à 9,5, de préférence de 7,5 à 9, en particulier de 8 à 8,5.

5. Procédé selon l'une des revendications 1 à 4 **caractérisé en ce que** la solution aqueuse contient la diamine en une quantité de 5 à 400, de préférence de 10 à 200 µM, en particulier de 80 µM.

6. Procédé selon l'une des revendications 1 à 5 **caractérisé en ce que** l'incubation de la solution aqueuse avec l'échantillon a lieu à une température de 10 à 50°C, de préférence de 25 à 40°C, de préférence encore de 30 à 38°C, en particulier de 34 à 38°C.

7. Procédé selon l'une des revendications 1 à 6 **caractérisé en ce que** l'incubation de la solution aqueuse avec l'échantillon a lieu pendant 30 minutes à 36 heures, de préférence pendant 1 à 30 heures, de préférence encore pendant 3 à 24 heures, en particulier pendant 18 heures.

8. Procédé selon l'une des revendications 1 à 7 **caractérisé en ce que** l'incubation de la solution aqueuse avec l'échantillon est arrêtée avant la dérivatisation avec l'aminoguanidine.

9. Procédé selon l'une des revendications 1 à 8 **caractérisé en ce que** l'étape de procédé de dérivatisation comprend une acylation.

10. Procédé selon la revendication 9 **caractérisé en ce que** la diamine est acylée avec la NHS-biotine.

11. Procédé selon l'une des revendications 1 à 10 **caractérisé en ce que** la quantité de diamine dérivatisée est déterminée par chromatographie, en particulier par chromatographie liquide à haute performance (CLHP), électrophorèse, en particulier électrophorèse capillaire (EC), ou immunoanalyse, en particulier immunoanalyse enzymatique (ELISA ; Enzyme-linked Immnosorbent Assay) ou radio-immunoanalyse (RIA).

12. Procédé selon l'une des revendications 1 à 11 **caractérisé en ce qu'**une peroxydase, de préférence une catalase, est ajoutée à la solution aqueuse et/ou à l'échantillon.

13. Procédé selon la revendication 12 **caractérisé en ce que** la peroxydase est ajoutée en une quantité de 0,1 à 10, de préférence de 0,2 à 5, de préférence encore de 0,5 à 2, en particulier de 1 unité de peroxydase pour 100 µl d'échantillon.

14. Procédé selon l'une des revendications 1 à 13 **caractérisé en ce que**, avant le mélange et l'incubation de la diamine avec l'échantillon, la diaminooxydase dans l'échantillon est enrichie sélectivement à partir de l'échantillon au moyen d'un anticorps spécifique qui est lié à une phase solide.

15. Utilisation d'un ensemble comprenant :
- un réactif de dérivatisation de diamine,
- un moyen de détermination de diamine dérivatisée,
- une préparation de DAO stabilisée ayant une quantité définie de DAO,
- un tampon de DAO,
- éventuellement une peroxydase, de préférence une catalase, et
- éventuellement une diamine comme substrat, pour déterminer l'activité de la diaminooxydase (DAO ; EC. 1.4.3.6) dans un échantillon.

16. Utilisation selon la revendication 15 **caractérisée en ce que** le moyen de détermination de diamine dérivatisée comprend des anticorps dirigés contre la diamine dérivatisée, en particulier des anticorps polyclonaux, et éventuellement des anticorps secondaires, où au moins l'un de ces anticorps est marqué de préférence enzymatiquement ou chimiquement.
